# FASCICULE DE BREVET EUROPEEN

(11) **EP 2 398 756 B1**
(45) Date de publication et mention de la délivrance du brevet: **18.07.2018**
(21) Numéro de dépôt: 10708243.0
(22) Date de dépôt: 18.02.2010
(51) Int. Cl.: C07C 37/68, C07C 39/11, A23L 3/349, A23L 33/105, A61K 8/97, A61K 8/34, A61Q 19/02, A61Q 19/08

(54) **PROCEDE D'EXTRACTION DE COMPOSES PHENOLIQUES A PARTIR D'EAU DE VEGETATION DE L'OLIVE ET PREPARATION D'EXTRAIT TITRE EN POLYPHENOLS D'OLIVE ET DE RAISIN**
VERFAHREN ZUM EXTRAHIEREN PHENOLISCHER VERBINDUNGEN AUS OLIVENFRUCHTWASSER UND HERSTELLUNG EINES MIT OLIVEN- UND WEINTRAUBENPOLYPHENOLEN TITRIERTEN EXTRAKTS
METHOD FOR EXTRACTING PHENOLIC COMPOUNDS FROM OLIVE FRUIT WATER AND PREPARATION OF AN EXTRACT TITRATED WITH OLIVE AND GRAPE POLYPHENOLS

(30) Priorité: 18.02.2009 FR 0900752
(43) Date de publication de la demande: 28.12.2011
(73) Titulaire: Centre de Coopération Internationale en Recherche Agronomique pour le Développement (CIRAD), 75016 Paris (FR); Grap'sud, 30360 Cruviers-Lascours (FR); Institut National d'Etudes Superieures Agronomiques de Montpellier (Montpellier SupAgro), 34000 Montpellier (FR)
(72) Inventeur: URBAN, Nelly, 30190 Sainte Anastasie (FR); DORNIER, Manuel, 34830 Clapiers (FR); PALLET, Dominique, 34398 Montpellier (FR); REYNES, Max, 30830 Clapiers (FR)
(74) Mandataire: Gevers & Orès
(86) Numéro de dépôt international: PCT/FR2010/000137
(87) Numéro de publication internationale: WO 2010/094860

(56) Documents cités:
- EP-A1- 1 623 960
- EP-A1- 1 844 666
- WO-A1-2005/123603
- WO-A1-2006/005986
- ES-A1- 2 177 457
- FR-A1- 2 772 235
- US-A1- 2007 077 279
- SKALTSOUNIS L ET AL: "MINOS PROJECT Process development for an integrated olive mill waste management recovering natural antioxidants and producing organic fertilizer", INTERNET CITATION, [Online] XP002354663, internet Extrait de l'Internet: URL:http://www.pharm.uoa.gr/minos/manualen g.pdf> [extrait le 2005-11-16]

## Description

La présente invention se rapporte à un procédé d'extraction de composés phénoliques de faible masse moléculaire à partir d'eaux de végétation d'olive ; elle se rapporte également à des compositions enrichies en composés phénoliques comprenant au moins 40% de matière sèche, ladite matière sèche étant composée d'au moins 30% d'hydroxytyrosol ; l'invention se rapporte en outre à un procédé de séchage desdites compositions par atomisation en utilisant des extraits de polyphénols de raisin comme support de séchage ainsi qu'aux poudres titrées en composés phénoliques d'olive et de raisin obtenues après séchage.

Le procédé de fabrication de l'huile d'olive consiste à écraser (broyage) les olives entières jusqu'à l'obtention d'une pâte fine ou pulpe. Au cours de cette étape, le broyat d'olives peut être constamment lavé avec de l'eau. La pâte est ensuite pressée mécaniquement (pressurage) pour en extraire son contenu liquide. Le liquide obtenu est alors décanté pour séparer les deux phases qui le composent : l'huile et la phase aqueuse. Les eaux de lavage du broyat et la phase aqueuse récupérées à l'issue du pressage sont appelées eaux de végétation ou encore margines. Ces eaux représentent environ 60% du poids de l'olive.

Ces eaux de végétation sont très riches en composés phénoliques antioxydants ; un composé phénolique antioxydant particulièrement recherché est l'hydroxytyrosol ; il est présent à hauteur de 0,1 à 2 g/l dans les margines.

L'hydroxytyrosol est un composé phénolique simple ayant une chaîne latérale de type alcool en position 1 du cycle aromatique, ainsi que deux substitutions hydroxyles en positions 3 et 4 dudit cycle. Ce composé appartient à la famille des *ortho*-diphénols, composés généralement connus pour avoir des propriétés antioxydantes intéressantes.

Sa consommation participerait à l'effet bénéfique du régime crétois sur la santé humaine. De nombreuses recherches effectuées sur l'hydroxytyrosol ont montré l'intérêt de ce composé en tant qu'antioxydant. L'activité antioxydante de l'hydroxytyrosol lui confère des propriétés d'agent conservateur des corps gras ; il est également connu pour ses propriétés anti-bactériennes, anti-virales et anti-fongiques.

Ce composé est donc considéré comme un très bon complément ou additif alimentaire et comme un agent actif utile pour renforcer la protection cellulaire vis-à-vis du stress oxydant et des pathologies qu'il engendre.

En raison de l'intérêt, notamment pour les industries alimentaire et cosmétique, que représentent l'hydroxytyrosol et, plus généralement, les polyphénols d'olives, diverses tentatives de valorisation des sous-produits riches en composés phénoliques tels que les eaux de végétation (Visioli et al. « Waste waters from olive oil production are rich in natural antioxidants » Experientia, 1995; 51: 32-34) ont été menées.

Le Brevet Américain US 6,361,803 décrit ainsi un procédé d'extraction de compositions antioxydantes à partir de produits dérivés d'olives : la pulpe, l'huile ou encore les eaux de végétation d'olives à différents stades de maturité. Lorsque le produit de départ est constitué d'eaux de végétation, le procédé consiste à les traiter sur une résine polymérique adsorbante qui piège les composés antioxydants, puis à laver la résine avec un solvant polaire organique pour récupérer les composés antioxydants.

La mise en oeuvre de ce procédé apparait cependant moins adaptée aux eaux de végétation qu'aux autres produits dérivés d'olive. En effet, la comparaison de la composition en composés phénoliques d'extraits obtenus à partir de différents matériaux de départ montre que celui obtenu à partir d'eaux de végétation présente une faible teneur en composés phénoliques : 16,8% ; en outre, cet extrait a une activité antioxydante la plus faible de tous les extraits obtenus.

La Demande de Brevet Européen EP 1 623 960 décrit un procédé de préparation de tyrosol et/ou d'hydroxytyrosol à partir d'eaux de végétation d'olive (margines) qui comprend des étapes de :
- séparation physique impliquant plusieurs traitements membranaires (microfiltration, ultrafiltration, nanofiltration et osmose inverse) des margines, de préférence à un pH neutre ou alcalin ;
- séparation chromatographique du tyrosol, de l'hydroxytyrosol et d'autres composés phénoliques à partir du concentrat obtenu après le dernier traitement membranaire ;
- conversion catalytique du tyrosol en hydroxytyrosol en présence d'un mélange de trioxyde de méthylrhénium (MTO) et de peroxyde d'hydrogène ;
- récupération de la phase aqueuse comprenant les composés phénoliques antioxydants.

Ce procédé présente l'inconvénient de mettre en oeuvre une hydroxylation chimique du tyrosol en hydroxytyrosol ; or, en vue de la consommation ultérieure de l'hydroxytyrosol obtenu, il est nécessaire d'éliminer les catalyseurs, notamment le trioxyde de méthylrhénium qui est un fort irritant. En outre, ce procédé comporte de très nombreuses étapes : l'exemple 1 de ce document met en oeuvre un procédé qui ne comporte pas moins de 13 traitements membranaires. Il est donc très long à mettre en oeuvre, coûteux en moyens techniques et humains et son dimensionnement à l'échelle industrielle n'est pas économiquement envisageable.

Il en est de même du procédé décrit dans la Demande Internationale WO 2005/123603 qui est destiné au fractionnement des eaux de végétation d'olives dans le but de les valoriser. Ce procédé comprend une étape d'ajustement du pH ; d'hydrolyse enzymatique de la cellulose, de l'hémicellulose et de la pectine contenues dans les eaux de végétation ; de centrifugation ; de microfiltration tangentielle ; d'ultrafiltration tangentielle ; de diafiltration ; de nanofiltration tangentielle et d'osmose inverse.

La Demande Internationale WO 2007/013032 décrit une méthode d'obtention de concentrats riches en hydroxytyrosol notamment à partir d'eaux de végétation ; cette méthode combine au moins deux étapes :
- une extraction d'hydroxytyrosol et d'autres composés bioactifs à l'aide d'un fluide supercritique et/ou par nanofiltration ; et
- une osmose inverse.

L'extraction par fluide supercritique nécessite l'utilisation de pressions très élevées (supérieures à 74 bars lors de l'utilisation de CO₂ comme fluide supercritique), c'est donc un procédé complexe et également coûteux à mettre en oeuvre. En outre, l'osmose inverse est un procédé qui conduit à la concentration d'un extrait par élimination d'eau ; en particulier, l'osmose inverse ne permet pas un enrichissement sélectif en composés phénoliques de bas poids moléculaire.

Le Brevet Américain US 6,416,808 décrit un procédé d'obtention d'une composition riche en hydroxytyrosol à partir d'eaux de végétation d'olive comprenant les étapes suivantes : (i) production d'une eau de végétation à partir d'olives dénoyautées ; (ii) ajout d'une quantité suffisante d'acide, de préférence de l'acide citrique, pour atteindre un pH compris entre 1 et 5 ; (iii) incubation des eaux de végétation acidifiées pendant au moins deux mois afin que l'oleuropéine soit hydrolysée en hydroxytyrosol. Le procédé peut en outre comprendre une étape d'extraction de l'hydroxytyrosol avec un solvant organique, une chromatographie en phase liquide à haute pression (HPLC) ou encore une extraction par fluide supercritique.

Bien qu'il conduise à des compositions enrichies en hydroxytyrosol, ce procédé, lorsqu'il met en oeuvre une extraction par HLPC ou fluide supercritique, est trop coûteux pour être utilisé à l'échelle industrielle. En outre, l'extraction liquide-liquide utilise des solvants organiques toxiques qui ne sont pas compatibles avec une utilisation de l'extrait obtenu dans les industries cosmétique ou alimentaire.

Ainsi les procédés décrits dans l'art antérieur ne donnent pas entière satisfaction et il demeure le besoin de mettre au point un procédé d'extraction de composés phénoliques de faible masse moléculaire à partir d'eaux de végétation de l'olive qui soit simple et adapté à une mise en oeuvre industrielle.

La Demanderesse s'est fixée comme objectif de proposer un procédé conduisant à un fractionnement des phénols contenus dans les margines afin de séparer la fraction phénolique de faible masse moléculaire des composés de masse moléculaire plus élevée comme l'oleuropéine et le verbascoside.

Pour présenter un intérêt dans le cadre de la présente invention, il est nécessaire que le procédé permette un enrichissement sélectif en hydroxytyrosol et en tyrosol. C'est ce à quoi est parvenue la Demanderesse en sélectionnant des membranes de nanofiltration qui présentent une rétention en hydroxytyrosol et en tyrosol la plus faible possible et, simultanément, une rétention maximale des autres composés phénoliques, en particulier des composés polyphénoliques. Ainsi, l'utilisation des membranes de nanofiltration sélectionnées permet d'augmenter de 1,5 à 3,5 fois la teneur massique en composés phénoliques de la matière sèche des perméats et, en particulier, 2 à 20 fois la teneur massique en hydroxytyrosol de la matière sèche des perméats issus du traitement de nanofiltration ; les perméats sont donc significativement enrichis en composés phénoliques de faible masse moléculaire.

La matière sèche d'un échantillon est mesurée par séchage de l'échantillon ; le pourcentage de matière sèche est alors déterminé par le ratio entre la masse de la matière sèche et la masse totale de l'échantillon.

A moins qu'il n'en soit précisé autrement, dans ce qui suit, les concentrations sont exprimées en pourcentages en masse.

On entend par rétentat, la fraction d'un échantillon retenue par une membrane lors du traitement de séparation membranaire dudit échantillon.

On entend par perméat, la fraction d'un échantillon traversant une membrane lors du traitement de séparation membranaire dudit échantillon.

Par traitement de séparation membranaire, on entend dans le cadre de la présente invention, un procédé de filtration -microfiltration, ultrafiltration, nanofiltration- ou d'osmose inverse.

On entend par éluat, le produit obtenu par la remise en solution par un solvant d'une ou plusieurs substances adsorbées sur un support ; l'éluat comprend donc le solvant et la ou lesdites substances.

Par eaux de végétation ou margines, on entend les eaux générées lors de la production de l'huile d'olive. La teneur en matière sèche des margines est variable et dépend du procédé mis en oeuvre en huilerie ; à titre indicatif, les margines brutes non clarifiées peuvent contenir entre 3 à 15% de matière sèche.

Selon un premier de ses objets, la présente invention se rapporte à un procédé selon la revendication 1.

Il est décrit un procédé d'extraction de composés phénoliques de faible masse moléculaire à partir d'eaux de végétation d'olives comprenant :
(a) une étape de clarification desdites eaux de végétation ;
(b) une étape d'ultrafiltration tangentielle des eaux de végétation clarifiées obtenues à l'étape (a) sur une membrane céramique tubulaire de seuil de coupure compris entre 100 et 200 kDa et de récupération du perméat ;
ledit procédé étant caractérisé en ce qu'il comprend en outre :
(c) une étape de nanofiltration dudit perméat obtenu à l'étape (b) sur une membrane organique spirale de seuil de coupure compris entre 150 et 400 Da à une pression comprise entre 1 et 3 MPa ; ladite membrane est telle qu'elle comporte une couche active comprenant au moins 50% de polyamide et qu'elle permet d'obtenir un rétentat comprenant au moins 70%, de préférence, entre 70 et 90% et, encore préférentiellement, au moins 80% de la matière sèche contenues dans lesdites eaux de végétation et au plus 25%, ou au plus 20%, d'hydroxytyrosol et
(d) une étape d'adsorption des composés phénoliques contenus dans le perméat obtenu à l'étape (c) par passage sur une matrice polymérique suivie de l'élution des composés phénoliques adsorbés sur ladite matrice avec un solvant polaire organique et obtention d'un éluat.

Le procédé selon l'invention présente l'avantage de conduire, avec uniquement deux étapes de traitement membranaire (ultrafiltration et nanofiltration), à un enrichissement sélectif en composés phénoliques de faible masse moléculaire. Ce procédé présente en outre l'originalité de permettre de séparer en différentes fractions des composés phénoliques des eaux de végétation : alors que les procédés connus concentrent l'ensemble des composés phénoliques des eaux de végétation à l'aide de traitements membranaires (les composés phénoliques sont alors récupérés dans le rétentat), dans le procédé selon l'invention, les membranes de nanofiltration utilisées permettent la séparation des composés phénoliques, les composés phénoliques d'intérêt étant alors récupérés dans le perméat.

Pour être utilisées dans le procédé selon l'invention, les eaux de végétation peuvent comprendre jusqu'à 15% de matière sèche. Le pourcentage de matière sèche des eaux de végétation est le ratio entre la masse de la matière sèche et la masse totale des eaux de végétation. En particulier, les eaux de végétation présentent un taux de matière sèche compris entre 3 et 7%. L'utilisation de margines comportant plus de 7% de matière sèche nécessite un traitement préalable tel qu'une dilution.

Par composés phénoliques de faible masse moléculaire, on entend des composés ayant une masse moléculaire inférieure ou égale à 400 Da. En particulier, les composés phénoliques de faible masse moléculaire sont l'hydroxytyrosol, le tyrosol, les acides phénoliques tels que les acides hydroxycinnamiques, comme par exemple les acides ferrulique, coumarique ou caféique, ou leurs esters, comme par exemple l'acide chlorogénique.

La clarification consiste en une extraction solide-liquide, telle que la filtration, la centrifugation ou encore la décantation, des eaux de végétation.

Par filtration, on entend un procédé de séparation des constituants d'un mélange qui possède une phase liquide et une phase solide au travers d'un milieu poreux. Pour la mise en oeuvre de l'étape (a) du procédé selon l'invention, on peut utiliser une filtration clarifiante, c'est-à-dire un milieu poreux tel que le diamètre des pores se situe entre 10 et 450 µm ; la filtration peut être mise en oeuvre à l'aide de filtres à cartouche, à bougies, à plateaux, à sable, de filtres presses ou de filtres rotatifs sous vide.

La centrifugation est une technique utilisant la force centrifuge pour séparer des éléments solides en suspension dans un fluide ; cette technique est mise en oeuvre à l'aide d'une centrifugeuse dont l'homme du métier choisira les caractéristiques en fonction des margines à traiter ; à titre d'exemple, on peut citer les centrifugeuses à bol tubulaire, à bol à chambres concentriques, les centrifugeuses à assiettes ou décanteur centrifuge.

Enfin, la décantation est une opération de séparation mécanique, sous l'action de la gravitation, de plusieurs phases non-miscibles dont l'une au moins est liquide. On peut ainsi séparer des solides insolubles en suspension dans un liquide comme dans le cas des margines. Pour mettre en oeuvre une telle décantation, on peut utiliser un décanteur (aussi appelé épaississeur) dont l'homme du métier ajustera les caractéristiques en fonction des margines à traiter, on peut notamment citer les décanteurs discontinus et les décanteurs continus éventuellement à cloison ou à étage.

De préférence, la clarification est réalisée par une opération de filtration.

L'ultrafiltration tangentielle mise en oeuvre à l'étape (b) du procédé selon l'invention est un procédé mécanique de séparation en phase liquide basé sur les propriétés de tamisage moléculaire d'une membrane poreuse balayée tangentiellement par un liquide contenant les constituants à séparer. Dans le cadre du présent procédé, l'ultrafiltration tangentielle est réalisée avec des membranes céramiques tubulaires ayant un seuil de coupure de préférence de 150 kDa comme par exemple les membranes d'ultrafiltration proposées par les sociétés TAMI et EXEKIA. Le procédé est mis en oeuvre à basse pression, c'est-à-dire inférieure ou égale à 0,1 MPa.

Après le passage des eaux de végétation clarifiées obtenues à l'étape (a) à travers la membrane d'ultrafiltration, on obtient deux fractions : le perméat qui contient le solvant et toutes les molécules dissoutes ayant un diamètre inférieur au diamètre des pores de la membrane ; et le rétentat qui est enrichi par rapport au liquide initial en molécules et composés ayant un diamètre supérieur au diamètre des pores de la membrane.

L'étape (c) consiste en un traitement de nanofiltration du perméat obtenu à l'étape (b). La nanofiltration est un procédé de séparation effectué par l'application d'une pression, qui en est la force motrice, sur une membrane semiperméable ; elle se caractérise par la nature de la membrane utilisée qui définit la taille des particules ciblées et la pression d'opération.

Dans le cadre du procédé selon la présente invention, l'étape de nanofiltration est réalisée sur une membrane organique spirale de seuil de coupure compris entre 150 et 400 Da, de préférence entre 300 et 400 Da, dont la couche active comprend au moins 50% de polyamide. Dans ces conditions, la nanofiltration du perméat issu de l'étape (b) conduit à un second perméat issu de l'étape (c) contenant les composés phénoliques de faible masse moléculaire (hydroxytyrosol, tyrosol et acides phénoliques).

De façon plus spécifique, il a été montré dans l'exemple 1 ci-après que seules les membranes organiques spirales de seuil de coupure compris entre 150 et 400 Da dont la couche active comprend au moins 50% de polyamide et présentant les caractéristiques décrites ci-après conduisent à un fractionnement satisfaisant des composés phénoliques des margines : ainsi, sont utiles selon l'invention les membranes dont l'utilisation conduit à un rétentat comprenant entre au moins 70%, de préférence, entre 70 et 90% et, encore préférentiellement, au moins 80%, de la matière sèche contenues dans lesdites eaux de végétation et au plus 25% ou au plus 20% d'hydroxytyrosol. Ces valeurs sont par exemple obtenues lors du traitement de margines à 4% de matière sèche dans le cadre d'essais sur installation pilote de laboratoire à 3 1 de volume nominal en configuration plane avec une surface membranaire utile de 1,5.10⁻² m⁻², une pression transmembranaire comprise entre 1 et 3 MPa et une vitesse tangentielle de 1 m.s⁻¹.

En outre, les membranes de nanofiltration utilisables dans le procédé de la présente invention sont préférentiellement sélectionnées parmi celles présentant une densité de flux de perméat d'au moins 10 L.h⁻¹.m⁻².

Les membranes de nanofiltration utilisables selon l'invention sont constituées :
(i) d'un matériau poreux organique qui joue un rôle de support assurant la résistance mécanique de la membrane ; ledit matériau poreux est en général composé de matériaux cellulosiques (par exemple, l'acétate de cellulose), des matériaux sulfonés, de matériaux polyamides et polyimides, de matériaux acryliques employés seuls ou sous forme de copolymères ou d'alliages de polymères, de matériaux fluorés, ou encore de polycarbonate et de polypropylène ; et
(ii) d'une couche active composée d'au moins 50% de polyamide qui recouvre ledit matériau poreux organique ; la couche active est composée de préférence d'au moins 70%, et encore préférentiellement d'au moins 90% de polyamide.

Par couche active, on entend la surface de la membrane de nanofiltration en contact avec l'échantillon auquel est appliqué le traitement de nanofiltration; en plus du polyamide, ladite couche active peut être composée, de façon non exhaustive, de polysulfones, de polyéthersulfones, de polyimides et/ou d'alcool polyvinylique.

Par polyamide, on entend un polymère résultant de la polycondensation de fonctions acide carboxylique et amine ; il s'agit plus particulièrement de polyamides aliphatiques et de polyamides aromatiques.

La mise au point de l'étape de nanofiltration a nécessité plusieurs essais sur diverses installations pilotes. Les résultats de sélection des membranes sont détaillés dans l'exemple 1.

Grâce à cette sélection de membranes, la fraction phénolique des perméats obtenus après nanofiltration est significativement enrichie en composés phénoliques de faible masse moléculaire ; à titre indicatif, l'hydroxytyrosol et le tyrosol y représentent entre 63 et 84% des composés phénoliques totaux.

De façon préférée, la mise en oeuvre de l'étape (c) de nanofiltration est réalisée dans des conditions telles que le flux de perméat est supérieur ou égal à 10 kg.h⁻¹.m⁻² (voir l'exemple 2 ci-après).

L'étape (d) de passage sur la matrice polymérique adsorbante permet d'éliminer les composés non phénoliques présents dans le perméat, notamment les minéraux, les sels et les acides organiques. Les composés phénoliques totaux représentent environ 30% de la matière sèche du perméat ; après passage sur résine, ils représentent plus de 80% de la matière sèche de l'éluat.

La matrice polymérique utilisée à l'étape (d) est de préférence une résine adsorbante styrénique, acrylique ou phénolique ; de préférence, il s'agit d'une résine adsorbante polystyrénique.

Les solvants polaires organiques ou la solution aqueuse de solvants polaires organiques utiles pour l'élution des composés phénoliques adsorbés sur ladite matrice sont notamment des solvants acceptables dans le domaine alimentaire ; de préférence, il s'agit d'un alcool en C₁-C₄ ou d'un mélange d'alcools ; préférentiellement, il s'agit de l'éthanol.

Le procédé selon l'invention comprend en outre une ou plusieurs des étapes suivantes :
- une étape optionnelle (a0), préalable à l'étape (a), d'acidification des eaux de végétation pour atteindre un pH inférieur ou égal à 4,5.

L'acidification des eaux de végétation peut être mise en oeuvre avec tout acide compatible avec la consommation alimentaire humaine ou animale. En particulier, l'acidification peut être réalisée avec tout acide organique tel que l'acide citrique, l'acide tartrique, l'acide malique ou l'acide lactique. Avantageusement, l'acidification est réalisée avec de l'acide citrique. Quel que soit l'acide utilisé pour la mise en oeuvre de l'étape (a0) du procédé selon l'invention, l'homme du métier saura ajuster la quantité nécessaire d'acide à introduire dans les eaux de végétation, par exemple par une méthode itérative consistant à ajouter des petites quantités d'acide puis à mesurer le pH.
- une étape (e) de concentration par distillation sous vide de l'éluat obtenu à l'étape (d).

Selon une variante particulière de mise en oeuvre du procédé, il comprend successivement les étapes (a0), (a), (b), (c), (d) et (e).

Le procédé peut encore comprendre trois étapes additionnelles utiles en vue d'un stockage des eaux de végétation avant la mise en oeuvre du procédé selon l'invention. Ces trois étapes, s'intercalant entre les étapes (a) et (b), sont les suivantes :
(a1) concentration des margines acidifiées et clarifiées pour les stocker ;
(a2) stockage des margines acidifiées concentrées ; puis
(a3) dilution dans un solvant des margines acidifiées concentrées à environ 4% de matière sèche avant de pratiquer l'étape (b).

L'étape (a1) peut être réalisée par une technique de concentration sous vide.

Le stockage selon l'étape (a2) est préférentiellement réalisé à une température comprise entre 6 et 15°C.

Le solvant de l'étape (a3) est de préférence de l'eau.

La mise en oeuvre du procédé décrit conduit à une composition enrichie en composés phénoliques de faible masse moléculaire. Cette composition -ci-après appelée composition A- comprend avantageusement au moins 40% de matière sèche ; ce pourcentage de matière sèche est le ratio entre la masse de la matière sèche obtenu après séchage de la composition A et la masse totale de la composition A ; ladite matière sèche est composée d'au moins 80% de composés phénoliques totaux ; en particulier, elle comprend au moins 30% d'hydroxytyrosol et au moins 10% de tyrosol.

Ce procédé présente en particulier l'avantage de conduire de façon reproductible à une composition en composés phénoliques comprenant des teneurs minimales en hydroxytyrosol, en tyrosol et en autres composés phénoliques de faible masse moléculaire tels que les acides hydroxycinnamiques, comme par exemple les acides ferrulique, coumarique ou caféique, ou leurs esters, comme par exemple l'acide chlorogénique. C'est un critère important pour une utilisation ultérieure dans les industries cosmétique ou alimentaire par exemple.

La composition A ainsi obtenue est séchée, par exemple par atomisation. De préférence, et afin d'éviter l'utilisation de supports de séchage tels que des polyosides de charge comme les dextrines ou les gommes, on utilise comme support de séchage un extrait de raisin comprenant au moins 60% en masse de polyphénols totaux. Ainsi, l'invention se rapporte également à l'utilisation d'un extrait de raisin comprenant au moins 60% en masse de polyphénols totaux comme support de séchage par atomisation d'une composition A.

L'invention se rapporte encore à un procédé de préparation d'une poudre titrée en composés phénoliques et polyphénoliques d'olive et de raisin selon la revendication 2. Il est décrit un procédé comprenant les étapes suivantes :
(e1) mélange d'une composition A avec un extrait de raisin comprenant au moins 60% de polyphénols totaux ; ledit mélange comprend une quantité de ladite composition A telle que la matière sèche de ladite composition A représente entre 5 et 40% de la matière sèche totale du mélange ;
(e2) séchage par atomisation du mélange obtenu à l'étape (e1).

Le pourcentage de matière sèche du mélange de la composition A et de l'extrait de raisin est le ratio entre la masse de la matière sèche mesurée après séchage dudit mélange et la masse totale dudit mélange.

Ledit extrait de raisin comprend de préférence au moins 60% de polyphénols totaux, et encore préférentiellement au moins 90% de polyphénols totaux. La teneur en polyphénols totaux d'un extrait de raisin peut notamment être mesurée par la méthode de Folin-Ciocalteu (Folin Singleton VL, Orthofer R, Lamuela-Raventos RM. Analysis of total phenols and other oxidation substrates and antioxidants by means of Folin-Ciocalteu reagent. Meth Enzymol 1999; 299: 152-178).

L'extrait de raisin peut être obtenu par passage sur une résine adsorbante, notamment une résine adsorbante styrénique, acrylique ou phénolique, de piquette de marc ou d'extraits aqueux de pépins de raisin. La piquette de marc est une solution obtenue après la diffusion à l'eau de marc de raisin ; le marc de raisin est la partie solide récupérée à l'issue de l'étape du pressage du raisin, il est constitué de l'ensemble des pellicules, des pépins de raisin ainsi que de la rafle.

Selon une variante de mise en oeuvre de ce procédé, ces deux étapes (e1) et (e2) suivent l'étape (e) du procédé d'extraction de composés phénoliques de faible masse moléculaire selon l'invention.

Le séchage par atomisation (ou pulvérisation) est une technique d'élimination d'eau par entraînement consistant à pulvériser un produit liquide dans un courant d'air chaud. Concrètement, cette technique consiste à injecter le produit liquide à déshydrater sous la forme d'un fin brouillard en haut d'une tour en même temps qu'un courant d'air très chaud parcourt ladite tour ; sous l'effet de la chaleur, l'eau s'évapore et la poudre déshydratée tombe en bas de la tour.

Ledit procédé de préparation d'une poudre titrée en composés phénoliques et polyphénoliques d'olive et de raisin conduit à une poudre comprenant au moins 95% de composés phénoliques et polyphénoliques.

Il est également décrit une poudre titrée en composés phénoliques et polyphénoliques d'olive et de raisin comprenant au moins 95% de composés phénoliques, dont 5 à 40% de la matière sèche provient de la composition A ; la matière sèche provenant de ladite composition A, étant composée d'au moins 80% de composés phénoliques totaux dont au moins 30% d'hydroxytyrosol et au moins 10% de tyrosol, ladite composition.

L'exemple 5 montre que les poudres selon l'invention présentent une très bonne activité anti-radicalaire. En effet, cette activité antiradicalaire est supérieure à la somme de l'activité antiradicalaire de la composition A et de celle d'extraits de raisin pris séparément ; démontrant un effet synergique de l'association de la composition A et des polyphénols d'extraits de raisin dans les poudres de l'invention.

A titre indicatif, les poudres titrées en composés phénoliques et polyphénoliques d'olive et de raisin obtenues à partir d'olive et d'extraits de pépins de raisin présentent les gammes de teneurs suivantes :

| | |
|---|---|
| Composés phénoliques totaux | de 60 à 100% |
| Procyanidines | de 18 à 50% |
| Hydroxytyrosol | de 1,5 à 16% |
| Tyrosol | de 0,5 à 8% |

Dans le cas où les polyphénols de raisins proviennent de marc de raisin, la poudre comprend les gammes de teneurs suivantes :

| | |
|---|---|
| Composés phénoliques totaux | de 60 à 100% |
| Procyanidines | de 9 à 25% |
| Anthocyanes | de 1,2 à 8% |
| Hydroxytyrosol | de 1,5 à 16% |
| Tyrosol | de 0,5 à 8% |

Les poudres obtenues selon ce procédé sont également des objets de la présente invention.

Les compositions A enrichies en composés phénoliques de faible masse moléculaire et les poudres selon l'invention présentent de nombreuses applications. Elles ont un intérêt particulièrement marqué dans le domaine de la cosmétique, pour leur utilisation comme agent de prévention du stress oxydant de la peau (derme et épiderme), comme agent anti-radicalaire, comme agent anti-âge ; mais aussi dans le domaine alimentaire notamment pour une utilisation comme compléments alimentaires ou encore comme conservateur.

Les compositions A enrichies en composés phénoliques de faible masse moléculaire et les poudres décrites ici sont particulièrement actives comme agents dépigmentants et/ou blanchissants de la peau ; elles favorisent ainsi l'élimination des taches et des irrégularités pigmentaires de la peau.

L'invention est maintenant décrite plus en détail au regard des exemples qui suivent. Il doit être bien entendu, toutefois, que ces exemples sont donnés uniquement à titre d'illustration de l'objet de l'invention, dont ils ne constituent en aucune manière une limitation.

### Exemple 1 - Sélection d'une membrane de nanofiltration

Afin d'optimiser la séparation des composés phénoliques de faible masse moléculaire par nanofiltration, plusieurs membranes commerciales ont été évaluées en termes de performance (densité de flux de perméat) et de rétention.

Les essais ont été réalisés à 30 ± 2°C sur une installation pilote de laboratoire (31 de volume nominal) en configuration plane avec une surface membranaire utile de l'ordre de 1,5.10⁻² m². Les pressions transmembranaires choisies s'échelonnent entre 1 et 3 MPa et la vitesse tangentielle avoisine 1 m.s⁻¹.

Pour évaluer la faisabilité de la séparation des composés phénoliques de faible masse moléculaire, neuf membranes de nanofiltration ont été présélectionnées (fournisseur Microdyn Nadir, Dow Filmtec, GE Osmonics, Toray, Koch). Ces membranes présentent un seuil de coupure compris entre 150 et 400 Da (valeurs annoncées par les fabricants).

Elles ont été testées sur une margine standard à 4% de matière sèche sans concentration, c'est-à-dire avec un facteur de réduction volumique proche de 1. Selon la membrane et la pression transmembranaire utilisée, les densités de flux de perméat varient entre 3 et 133 kg.h⁻¹.m⁻².

Les résultats obtenus sont présentés dans le Tableau I ci-après.

**Tableau I : Résultats de nanofiltration obtenus sur les différentes membranes testées.**

| **Membranes** | | | **Densité de flux** | **Rétention (%)** | | |
|---|---|---|---|---|---|---|
| Fournisseur | Nature polymère couche active | Référence | **de perméat** (L.h⁻¹.m⁻²) | Matière sèche | Polyphénols totaux | Hydoxytyrosol |
| Mycrodin Nadir | Polyéthersulfones | NP030 | 3-5 | 78 | 51 | 17 |
| | | NP010 | 13-23 | 52-73 | 41-46 | 9 |
| Dow Filmtec | Polyamides | NF270 | 20-56 | 90-96 | 58-59 | 26-43 |
| | | NF200 | 14-42 | 87-94 | 56-64 | 22-40 |
| | | NF90 | 3-19 | > 99 | 98 | 99 |
| GE Osmonics | Polyamides | DK | 20-92 | 92-99 | 78-82 | 1-40 |
| | | DL | 27-133 | 75-97 | 51-78 | 1-44 |
| Toray | alcool polyvinylique / polyamides | UTC60 | 14-45 | 90-94 | 56-66 | 22-39 |
| Koch | Polyamides | MPF34 | 7-17 | >99 | 93 | 87 |

Les membranes testées retiennent entre 52 et 99% de la matière sèche et entre 41 et 98% de polyphénols totaux des margines traitées (déterminé avec la méthode de Folin-Ciocalteu, Folin Singleton VL, Orthofer R, Lamuela-Raventos RM. Analysis of total phenols and other oxidation substrates and antioxidants by means of Folin-Ciocalteu reagent. Meth Enzymol 1999; 299: 152-178.).

La rétention en hydroxytyrosol, principal composé phénolique de faible masse moléculaire, est plus faible ; les membranes testées retiennent entre 1 et 44% de l'hydroxytyrosol présent dans les margines traitées, à l'exception des membranes Dow Filmtec NF90 et Koch MPF34.

Bien que les membranes soient *a priori* voisines, on observe que les performances globales du procédé (densité de flux de perméat) et les performances de la séparation (rétention) sont étroitement liées au choix de la membrane de nanofiltration.

Dans le cadre de l'application envisagée, les membranes les plus appropriées sont celles qui permettent d'obtenir à la fois une faible rétention en hydroxytyrosol, une rétention élevée en matière sèche et en polyphénols totaux et, enfin, une densité de flux de perméat élevée. Parmi les membranes testées, la plupart des membranes dont la couche active se compose de polyamides conduisent à des résultats intéressants. Celles dont la couche active est constituée de polyéthersulfones ne répondent que partiellement à ces critères.

### Exemple 2 - Influence de la concentration des margines sur l'efficacité de l'étape de nanofiltration

Afin d'évaluer l'impact de la concentration des margines sur la densité de flux de permeat et la rétention, des margines présentant une teneur en matière sèche variant de 3,5 à 22% ont été traitées. Pour ces essais, deux membranes parmi les plus performantes testées à l'exemple 1, sont choisies : GE Osmonics Desal DL et DK.

Comme dans l'exemple 1, les essais ont été réalisés à 30°C sur une installation pilote de laboratoire en configuration plane avec une surface membranaire utile de l'ordre de 1,5.10⁻²m². Les pressions transmembranaires utilisées s'échelonnent entre 20 bars (2 MPa) et 30 bars (3 Mpa) et la vitesse tangentielle avoisine 1 m.s⁻¹.

Les résultats obtenus sont présentés dans le Tableau II.

**Tableau II**

| Membrane | Matière sèche des margine (%) | Pression transmembranaire (bar) | Flux de perméat (kg.h⁻¹.m⁻²) |
|---|---|---|---|
| GE Osmonics Desal DL | 4 | 20 | 95,4 |
| | | 25 | 105,3 |
| | | 30 | 132,7 |
| | 13 | 20 | 10,4 |
| | | 25 | 19,5 |
| | | 30 | 29,8 |
| | 22 | 20 | < 0,8 |
| | | 25 | 1,1 |
| | | 30 | 3,2 |
| GE Osmonics Desal DK | 4 | 20 | 63,2 |
| | | 25 | 78,2 |
| | | 30 | 91,9 |
| | 13 | 20 | 5,0 |
| | | 25 | 12,7 |
| | | 30 | 22,5 |
| | 22 | 20 | < 0,8 |
| | | 25 | < 0,8 |
| | | 30 | 0,8 |

Pour les deux membranes, le flux de perméat diminue rapidement avec l'augmentation de la teneur en matière sèche. Par exemple, à 20 bars, la densité de flux de perméat est divisée par 10 lorsque la teneur en matière sèche passe de 4 à 13%. A 22% de matière sèche, les densités de flux de perméat sont très faibles : la pression doit être maintenue supérieure à 25 bars pour obtenir des densités de flux de perméat de quelques kg.h⁻¹.m⁻².

Ces résultats montrent que, pour garantir un flux supérieur à 10 kg.h⁻¹.m⁻² dans les conditions opératoires testées, il est difficile de dépasser 13 à 15% de matière sèche. Cette teneur correspond à un facteur de réduction volumique compris entre 3 et 4 soit un rendement de production de perméat voisin de 70%.

Le facteur de réduction volumique (FRV) est défini comme le rapport entre le volume de produit initial sur le volume de rétentat final soit FRV = V₀/Vr ; comme V₀ = Vr + Vp, FRV est directement lié au rendement volumique de production de perméat X = Vp/V0 soit en pourcentage X = 100.(1-1/FRV).

On constate par ailleurs que les rétentions sont peu affectées par la teneur en matière sèche des margines l'alimentation ; les taux de matière sèche et de polyphénols totaux dans le rétentat diminue légèrement lorsque la concentration des margines augmente.

### Exemple 3 - Essais de nanofiltration à une échelle semi-industrielle

Les essais de laboratoire ont permis d'évaluer la faisabilité technique du procédé de nanofiltration pour enrichir le produit en composés phénoliques de faible masse moléculaire. Ils ont également permis de sélectionner des membranes commerciales appropriées pour réaliser l'opération. Afin d'évaluer le procédé à une échelle plus en adéquation avec une utilisation industrielle, des essais ont été réalisés en utilisant des modules de nanofiltration spiralés de 2,5 m² de surface utile. L'installation mise en oeuvre a une capacité nominale de 801. La pression transmembranaire choisie est comprise entre 2 et 3 MPa, la température est de 30°C et le débit de circulation est de 1,7.10⁻⁴ m³.s⁻¹.

Le premier essai est réalisé sur les mêmes membranes que celles présentées en exemple 2, c'est-à-dire les membranes GE Osmonics Desal DL et DK. Lors de cet essai, le perméat est extrait continuellement jusqu'à atteindre un facteur de réduction volumique de 8.

Les résultats obtenus en termes de performance sont en adéquation avec ceux obtenus sur pilote de laboratoire : la densité de flux de perméat diminue lorsque le pourcentage de matière sèche des margines augmente. Le facteur de réduction volumique maximal pour obtenir une densité de flux de perméat supérieure à 10 kg.h⁻¹.m⁻² est compris entre 4,5 et 6 soit un rendement de production de perméat voisin de 80%, Pour des facteurs de réduction volumique compris entre 2 et 6, la matière sèche du perméat est enrichie de 2 à 4 fois en composés phénoliques totaux et de 5 à 13 fois en hydroxytyrosol.

Des essais complémentaires ont été réalisés avec les deux mêmes modules membranaires spiralés de 2,5 m² et dans des conditions similaires à celles décrites ci-avant. Ces essais visent à vérifier la stabilité des performances de l'opération en simulant un fonctionnement continu avec deux étages de concentration successifs. Le premier étage correspond à un facteur de réduction volumique de 2 (soit un rendement de production de perméat de 50%) et le second à un facteur de réduction volumique de 8 (soit un rendement de production de 75%).

Les densités de flux de perméat sont environ 15 fois plus faibles dans le second étage par rapport au premier. Vérifiées sur des durées de palier de 120 minutes, les densités de flux sont stables au cours du temps dans les deux étages. En termes de rétention, les résultats corroborent ceux obtenus précédemment. La mise en oeuvre industrielle du procédé en continu est donc aisément envisageable.

### Exemple 4 - Mise en oeuvre du procédé selon l'invention à une échelle industrielle

Afin de valider le procédé dans sa globalité, un essai a été réalisé à l'échelle industrielle en intégrant toutes les étapes du procédé selon l'invention.

Pour cet essai, 19,3 m³ de margines fraîches acidifiées avec de l'acide citrique à pH 4,2 ont été utilisées.

Ces margines sont tout d'abord clarifiées par filtration sur filtre rotatif sous vide puis traitées par ultrafiltration sur une installation munie de 37,2 m² de membranes céramiques tubulaires de seuil de coupure 150 kDa. Le perméat obtenu (13,9 m³) est ensuite nanofiltré sur une installation munie de 18 m² de membranes organiques spiralée GE Osmonics Desal DL. Le perméat de nanofiltration (10,6 m³) est alors traité sur des résines absorbantes RELITE SP411®. Après élution à l'éthanol à 80% et concentration sous vide de l'éluat alcoolique, 45,7 kg de composition enrichie en composés phénoliques de faible masse moléculaire à 48% de matière sèche sont obtenus. Cette composition contient 41,7% de polyphénols (A₂₈₀ₙₘ éq. Catéchine, c'est-à-dire exprimé en équivalent de la catéchine et mesuré avec un spectrophotomètre à 280 nm selon J. Zhishen, T. Mengcheng, W. Jianming (1999) The détermination of flavonoid contents in mulberry and their scavenging effects on superoxide radicals, J Food Chem, Volume 64, pp 555-559), 17% d'hydroxytyrosol et 6% de tyrosol (mesurés par chromatographie en phase liquide haute performance, HPLC).

La composition enrichie en composés phénoliques de faible masse moléculaire est enfin séchée par atomisation avec un extrait de raisin. Préalablement au séchage, la composition et l'extrait de raisin sont mélangés de telle sorte que la matière sèche du mélange est composée à 80% de matière sèche de raisin et 20% de matière sèche de ladite composition. Le produit fini obtenu est une poudre fluide qui ne prend pas en masse. Elle contient uniquement des polyphénols (100%, A₂₈₀ₙₘ éq. catéchine). Elle se constitue de 17,4% de procyanidines, 7,6% d'hydroxytyrosol, 2,7% de tyrosol (HPLC) et 3,1% d'anthocyanes (mesuré par la méthode de Riberau-Gayon et al., 1975, Sciences et techniques du vin, Tome III, Dunod, Paris).

Le séchage de ladite composition dans les mêmes conditions mais avec un support de polyphénols de pépins de raisin (de la même façon, le mélange est tel que la matière sèche du mélange est composée à 80% de matière sèche de pépins de raisin et 20% de matière sèche de la composition) conduit également à une poudre fluide, ne reprenant pas en masse et constituée uniquement de polyphénols. Cette seconde poudre se compose de 31,2% de procyanidines (méthode à la vanilline, Broadhurst R.B., Jones W.T. 1978. Analysis of condensed tannins using acidified vanillin. J. Science Food Agriculture, 29:788-794), de 6,9% d'hydroxytyrosol et de 2,5% de tyrosol (HPLC).

### Exemple 5 - Mesure de l'activité anti-radicalaire des compositions et poudres selon l'invention

Les propriétés anti-radicalaires des compositions enrichies en composés phénoliques de faible masse moléculaire obtenues à partir d'eaux de végétation et des poudres obtenues par séchage desdites compositions avec, comme support de séchage, des polyphénols de raisin ont été évaluées par la méthode ORAC.

La méthode ORAC, pour « Oxygen Radical Absorbance Capacity » (CAO et al. 1993, Free Radical Biol. Med. 14, 303-311 ; PRIOR et al. 2003, J. Agric. Food Chem. 51, 3273-3279) est une méthode de mesure *in vitro* de l'activité anti-radicalaire. Son principe est basé sur l'utilisation de la fluorescéine, molécule fluorescente naturelle très sensible aux radicaux libres. Les radicaux libres, qui sont produits par le AAPH (2,2'-Azobis (2-AmidinoPropane) diHydrochloride) dans le milieu réactionnel, réagissent avec les antioxydants de la composition ou la poudre à tester. Dès lors que tous les radicaux libres ont réagi avec les antioxydants la composition ou la poudre à tester, ils détruisent la fluorescéine et la fluorescence disparaît. La valeur ORAC est déterminée par la mesure de l'aire sous la courbe de la fluorescence en fonction du temps. Les résultats sont comparés aux données obtenues avec du TROLOX (analogue de la vitamine E) ; ils sont exprimés en micromoles TROLOX Equivalents (TEQ) par g, kg ou par litre.

Les valeurs ORAC mesurées dans le cadre de ces essais figurent dans le Tableau III.

**Tableau III**

| **Composition** | **Valeur ORAC de la composition** | **Valeur ORAC de la matière sèche** |
|---|---|---|
| composition enrichie en composés phénoliques de faible masse moléculaire d'eaux de végétation | 6 340 micromoles TEQ/g | 13200 micromoles TEQ/g |
| Polyphénols d'un extrait de pépin de raisin | - | 19000 micromoles TEQ/g |
| Polyphénols d'un extrait de raisin | - | 13100 micromoles TEQ/g. |
| poudre obtenue avec, comme support de séchage, l'extrait de pépin de raisin | 19300 micromoles TEQ/g | - |
| poudre obtenue avec, comme support de séchage, l'extrait de raisin | 15100 micromoles TEQ/g | - |

Pour une composition enrichie en composés phénoliques de faible masse moléculaire à partir d'eaux de végétation, la valeur ORAC est de 6340 micromoles TEQ/g ; sachant que la matière sèche de cet extrait est de 48%, cela correspond à 13200 micromoles TEQ/g de matière sèche.

La valeur ORAC de l'extrait de pépin de raisin et celle de l'extrait de raisin sont respectivement de 19000 micromoles TEQ/g et 13100 micromoles TEQ/g.

Ainsi, pour la poudre contenant 80% de matière sèche d'extrait de pépins de raisin et 20% de matière sèche extrait d'olive, on devrait s'attendre à une valeur ORAC théorique calculée à partir des valeurs ORAC des matières sèches correspondantes de 17840 micromoles TEQ/g ; or la mesure de la valeur ORAC est de 19300 micromoles TEQ/g.

De même, pour le mélange contenant 80% de matière sèche d'extrait de raisin et 20% de matière sèche extrait d'olive, on devrait observer une valeur ORAC de 13120 micromoles TEQ/g et la valeur mesurée est de 15100 micromoles TEQ/g.

Les valeurs ORAC obtenues sur les poudres selon l'invention sont donc supérieures à celles attendues de 8 à 15 % ce qui montre un effet synergique, au niveau de l'activité anti-radicalaire, de l'association de composés phénoliques de faible masse moléculaire de l'olive et des polyphénols de raisin.

### Exemple 6 - Exemple comparatif de mise en oeuvre du procédé selon l'invention à une échelle industrielle avec une membrane de nanofiltration ne présentant pas les caractéristiques requises

Un autre essai de mise en oeuvre du procédé à l'échelle industrielle est réalisé avec une membrane KOCH SR3 N2.

Pour cet essai, 28 m³ de margines sont acidifiées avec de l'acide citrique pour atteindre un pH 4,2.

Les margines acidifiées sont ensuite ultrafiltrées sur une installation munie de 37,2 m² de membranes céramiques tubulaires de seuil de coupure de 150 kDa.

Le perméat obtenu (24 m³) est nanofiltré sur une installation munie de 18 m² de membranes organiques spiralées KOCH SR3 N2. Le perméat de nanofiltration (18 m³) a été traité sur une résine adsorbante RELITE SP411. Après dilution à l'éthanol à 80% et concentration sous vide de l'éluat alcoolique, 41 kg de composition enrichie en composés phénoliques de faible masse moléculaire à 48,8% de matière sèche sont obtenus. Cette composition contient 37,6% de polyphénols (A₂₈₀ₙₘ éq. catéchine), 11% d'hydroxytyrosol et 4% de tyrosol (HPLC). Dans cette composition, le ratio hydroxytyrosol / polyphénols totaux est seulement de 29,3 g / 100 g. Cet essai confirme les résultats de l'exemple 1, la sélectivité de cette membrane de nanofiltration est insuffisante pour obtenir une composition enrichie en composés phénoliques d'intérêt.

## Revendications

1. Procédé de préparation de composition enrichie en composés phénoliques de masse moléculaire inférieure ou égale à 400 Da à partir d'eaux de végétation d'olives **caractérisé en ce qu'**il consiste en :
(a0) une étape optionnelle d'acidification des eaux de végétation pour atteindre un pH inférieur ou égal à 4,3 ;
(a) une étape de clarification desdites eaux de végétation par filtration, centrifugation ou décantation ;
optionnellement les trois étapes (a1) à (a3) suivantes :
(a1) la concentration des margines acidifiées et clarifiées pour les stocker ;
(a2) le stockage des margines acidifiées concentrées ; puis
(a3) la dilution dans un solvant des margines acidifiées concentrées à environ 4% de matière sèche avant de pratiquer l'étape (b) ;
(b) une étape d'ultrafiltration tangentielle des eaux de végétation clarifiées obtenues à l'étape (a) sur une membrane céramique tubulaire de seuil de coupure compris entre 100 et 200 kDa et de récupération du perméat ;
(c) une étape de nanofiltration dudit perméat obtenu à l'étape (b) sur une membrane organique spirale de seuil de coupure compris entre 150 et 400 Da à une pression comprise entre 1 et 3 MPa ; ladite membrane est telle qu'elle comporte une couche active comprenant au moins 50% de polyamide et qu'elle permet d'obtenir un rétentat comprenant au moins 80% de la matière sèche contenue dans lesdites eaux de végétation et au plus 25% d'hydroxytyrosol ;
(d) une étape d'adsorption des composés phénoliques contenus dans le perméat obtenu à l'étape (c) par passage sur une matrice polymérique suivie de l'élution des composés phénoliques adsorbés sur ladite matrice avec un solvant polaire organique et obtention d'un éluat ;
(e) une étape de concentration par distillation sous vide de l'éluat obtenu à l'étape (d) conduisant à un concentré dénommé composition A ;
et une étape de séchage de ladite composition A.

2. Procédé de préparation d'une poudre titrée en composés phénoliques et polyphénoliques d'olive et de raisin comprenant les étapes suivantes:
(e1) le mélange de la composition A obtenue par le procédé selon la revendication 1 avec un extrait de raisin comprenant au moins 60% de polyphénols totaux ; ledit mélange comprend une quantité de ladite composition telle que la matière sèche de ladite composition représente entre 5 et 40% de la matière sèche totale du mélange ; et
(e2) le séchage par atomisation du mélange obtenu à l'étape (e1).

## Patentansprüche

1. Verfahren zur Herstellung einer mit Phenolverbindungen angereicherten Zusammensetzung mit einem Molekulargewicht von maximal 400 Da aus Olivenfruchtwasser, **dadurch gekennzeichnet, dass** es besteht aus:
(a0) einem optionalen Schritt zur Ansäuerung des Fruchtwassers auf einen pH-Wert von maximal 4,3;
(a) einem Schritt zur Klärung dieses Fruchtwassers durch Filtration, Zentrifugation oder Dekantierung;
optional den folgenden drei Schritten (a1) bis (a3):
(a1) der Konzentration des angesäuerten und geklärten Fruchtwassers für die Lagerung;
(a2) die Lagerung des angesäuerten konzentrierten Fruchtwassers; dann
(a3) Verdünnen des konzentrierten angesäuerten Fruchtwassers auf etwa 4% Trockenmasse in einem Lösungsmittel, bevor Schritt (b) durchgeführt wird;
(b) einen Schritt zur Querstrom-Ultrafiltration des in Schritt (a) erhaltenen geklärten Fruchtwassers auf einer röhrenförmigen Keramikmembran mit einer Ausschlussgrenze zwischen 100 und 200 kDa und Permeatrückgewinnung;
(c) einen Schritt zur Nanofiltrierung des in Schritt (b) erhaltenen Permeats auf einer organischen Spiralmembran mit einer Ausschlussgrenze zwischen 150 und 400 Da bei einem Druck zwischen 1 und 3 MPa; wobei die Membran so beschaffen ist, dass sie eine aktive Schicht umfasst, die mindestens 50 % Polyamid umfasst, und dass sie es ermöglicht, ein Retentat zu erhalten, das mindestens 80 % der im Fruchtwasser enthaltenen Trockenmasse und höchstens 25 % Hydroxytyrosol enthält;
(d) einen Schritt zur Adsorbtion der in dem in Schritt (c) erhaltenen Permeat enthaltenen phenolischen Verbindungen durch Überqueren einer Polymermatrix, gefolgt von einer Elution der an der Matrix adsorbierten phenolischen Verbindungen mit einem organischen polaren Lösungsmittel und Erhalten eines Eluats;
(e) einen Schritt zur Konzentration durch Vakuumdestillation des in Schritt (d) erhaltenen Eluats, die zu einem Konzentrat mit der Bezeichnung Zusammensetzung A führt;
und einem Schritt zur Trocknung der Zusammensetzung A.

2. Verfahren zur Herstellung eines mit phenolischen und polyphenolischen Verbindungen titrierten Pulvers aus Oliven und Trauben, das die folgenden Schritte umfasst:
(e1) Mischen der durch das Verfahren nach Anspruch 1 erhaltenen Zusammensetzung A mit einem Traubenextrakt, der insgesamt mindestens 60 % Polyphenole enthält;
wobei die Mischung eine solche Menge der Zusammensetzung umfasst, dass die Trockenmasse der Zusammensetzung zwischen 5 % und 40 % der gesamten Trockenmasse der Mischung ausmacht; und
(e2) Sprühtrocknen der in Schritt (e1) erhaltenen Mischung.

## Claims

1. Preparation process of a composition enriched in phenolic compounds with a molecular weight of 400 Da or less from olive vegetation water, **characterised in that** it consists of:
(a0) an optional acidification step of the vegetation water to achieve a pH of 4.3 or less;
(a) a clarification step of said vegetation water by filtration, centrifugation or decantation;
optionally, the three following steps (a1) to (a3):
(a1) the concentration of the acidified and clarified amurca for storage;
(a2) the storage of concentrated acidified amurca; then
(a3) the dilution in a solvent of the acidified amurca concentrated to approximately 4% of dry matter before performing step (b);
(b) a tangential ultrafiltration step of the clarified vegetation water obtained in step (a) on a tubular ceramic membrane with a cut-off threshold ranging from 100 to 200 kDa and recovery of the permeate;
(c) a nanofiltration step of said permeate obtained in step (b) on a spiral organic membrane with a cut-off threshold ranging from 150 to 400 Da at a pressure ranging from 1 to 3 MPa; said membrane is such that it comprises an active layer that comprises at least 50% polyamide and it enables to obtain a retentate comprising at least 80% of the dry matter contained in said vegetation water and a maximum of 25% of hydroxytyrosol;
(d) an adsorption step of the phenolic compounds contained in the permeate obtained in step (c) by passage on a polymer matrix followed by elution of the phenolic compounds adsorbed onto said matrix with an organic polar solvent and obtainment of an eluate;
(e) a concentration step by vacuum distillation of the eluate obtained in step (d), leading to a concentrate called composition A;
and a drying step of said composition A.

2. A preparation process of a powder titrated with phenolic and polyphenolic compounds of olive and grape including the following steps:
(e1) the mixture of the composition A achieved by the process according to claim 1 with a grape extract featuring at least 60% total polyphenols; said mixture includes a quantity of said composition such that the dry matter of said composition represents between 5 and 40% of the total dry matter of the mixture; and
(e2) the mixture obtained in step (e1) is spray-dried.
